# EUROPEAN PATENT SPECIFICATION

(11) **EP 0 789 590 B1**
(45) Date of publication and mention of the grant of the patent: **04.09.2002**
(21) Application number: 95934516.6
(22) Date of filing: 25.09.1995
(51) Int. Cl.: A61K 47/48, A61K 39/00

(54) **CHITOSAN INDUCED IMMUNOPOTENTIATION**
CHITOSAN INDUZIERTE VERSTÄRKUNG
IMMUNOSTIMULATION INDUITE PAR LE CHITOSANE

(30) Priority: 23.09.1994 US 311532
(43) Date of publication of application: 20.08.1997
(73) Proprietor: ZONAGEN, INC., The Woodlands, TX 77380 (US)
(72) Inventor: PODOLSKI, Joseph, S., The Woodlands, TX 77381 (US); HSU, Kuang, The Woodlands, TX 77381 (US); BHOGAL, Balbir, The Woodlands, TX 77381 (US); SINGH, Gurpreet, Houston, TX 77059 (US)
(74) Representative: Walton, Seán Malcolm
(86) International application number: US9512189
(87) International publication number: WO96009805

(56) References cited:
- EP-A- 0 183 556
- EP-A- 0 308 147
- EP-A- 0 454 898
- WO-A-84/00294
- US-A- 4 935 147
- US-A- 4 939 239
- CHEMICAL ABSTRACTS, vol. 117, no. 3, 20 July 1992 Columbus, Ohio, US; abstract no. 24394, MARCINKIEWICZ, JANUSZ ET AL: "Immunoadjuvant properties of chitosan" XP002005132 cited in the application & ARCH. IMMUNOL. THER. EXP. (1991), 39(1-2), 127-32 CODEN: AITEAT;ISSN: 0004-069X, 1991,
- DATABASE CHEMABS CHEMICAL ABSTRACTS SERVICE, COLUMBUS, OHIO, US AN=101:83701, KASHKIN, K. P. ET AL: "Some properties of chitosan as a carrier in conjugated antigens" XP002005133 & IMMUNOLOGIYA (MOSCOW) (1984), (2), 31-5 CODEN: IMUNDA;ISSN: 0206-4952, 1984,
- DATABASE CHEMABS CHEMICAL ABSTRACTS SERVICE, COLUMBUS, OHIO, US AN=108:210053, TOKURA, S. ET AL: "Immunological aspects of chitin derivatives" XP002005134 & PROG. BIOTECHNOL. (1987), 3(IND. POLYSACCHARIDES), 347-62 CODEN: PBITE3, 1987,
- PATENT ABSTRACTS OF JAPAN vol. 017, no. 135 (C-1037), 19 March 1993 & JP,A,04 311385 (KYOWA HAKKO KOGYO CO LTD), 4 November 1992,
- DATABASE WPI Section Ch, Week 8717 Derwent Publications Ltd., London, GB; Class B04, AN 87-118805 XP002005135 & JP,A,62 061 927 (IHARA CHEM IND) , 18 March 1987

## Description

The present invention relates generally to compositions for potentiating an immune response in an animal, methods to produce the compositions and use of the compositions in the manufacture of a medicament for potentiating an immune response in an animal. More specifically, the invention provides adjuvants consisting essentially of, in combination, chitosan with a chelated metal, immunogens consisting essentially of an antigen in combination with the adjuvant, methods of making the adjuvant or immunogen, and use of the immunogen in the manufacture of a medicament for potentiating an immune response in an animal.

### BACKGROUND OF THE INVENTION

Recent biotechnological advances have facilitated identification of components in complex antigens which provide hope for successful development of safe and practical vaccines. Often, however, these isolated select components are not as immunogenic as the complete complex antigens from which they were derived. In order to enhance an immune response to the weakly antigenic immunogen in a recipient animal, adjuvants are frequently administered with the immunogen. Despite the universal acceptance of adjuvants, however, the number suitable for use in humans is limited.

Ideally, an adjuvant should potentiate long-lasting expression of functionally active antibodies, elicit cell-mediated immunity (CMI), and enhance production of memory T- and B-lymphocytes with highly specific immunoreactivity against an invading antigen. In addition to providing a defense upon immediate challenge with an foreign antigen, these responses should provide protection against any future encounters of the host with a specific antigen. More important is the ability of an adjuvant to augment the immune response with a minimum of toxic side effects. Therefore, efficacy of an adjuvant is described in terms of how it balances positive (potentiated immunity) and negative (toxicity) influences.

Controlled immunization for the purpose of stimulating antibody production by B cells is dependent upon a myriad of factors inherent to both the antigen itself and the immunized animal. In general, the farther removed in evolutionary terms the antigen, or its source, is from the invaded host, the more effective the immune response elicited by the antigen. Antigens derived from closely related species are less competent in eliciting antibody production due to the fact that the host immune system is unable to clearly distinguish the foreign antigen from endogenous, or self antigens. In addition, the dosage of the antigen, the purity of the antigen, and the frequency with which the antigen is administered are also factors which significantly contribute to the resulting antibody titer and specificity of the resulting antibodies. Still other factors include the form, or complexity, of the antigen, and how the antigen is administered. Finally, both the genetic makeup and overall physiological state of the immunized animal contribute to the extent to which an immune response is mounted. Of these factors, the form or complexity of the antigen is directly affected by immunization with an adjuvant.

Current understanding suggests that adjuvants act to augment the immune response by a variety of different mechanisms. In one mechanism, the adjuvant directly stimulates one of either CD4⁺ helper T-cell subpopulations designated T_{H}1 or T_{H}2 [Mosmann and Coffman, *Ann.Rev.Immunol. 7*:145-173 (1989)]. Helper T cells are required for B-cell antibody responses to most antigens. In an appropriate immune response, an antigen is captured and processed by an antigen-presenting cell (APC), *e.g*., circulating or tissue macrophages, and presented on the surface of the APC in association with a class II major histocompatibility (MHC) molecule. In this form, the antigen can interact with receptors on the surface of helper T cells thereby activating the particular subpopulation of cells to express and secrete any of a number of cytokines. The nature of cytokine production depends on the subset of helper T cells activated, a result that can be modulated in part by the choice of adjuvant. For example, alum, an aluminum salt adjuvant approved for clinical use in humans, has been reported to selectively activate T_{H}2 cells in mice [Grun and Maurer, *Cell.Immunol. 121*:134-145 (1989)], while Freund's complete adjuvant (FCA), an emulsion of mineral oil with killed mycobacteria [Freund, *et al., Proc.Soc.Exp.Biol.Med. 37*:509(1937)], preferentially activates murine T_{H}1 cells [Grun and Maurer, *Cell.Immunol. 121*:134-145 (1989)].

Another mechanism by which the immune response is augmented involves the direct stimulation of B cells by, for example, lipopolysaccharide (LPS) from Gram-negative bacteria. [Gery, *et al., J.Immunol. 108*:1088 (1972)]. LPS has also been shown to stimulate secretion of interferon-γ (INF-γ) [Tomai and Johnson, *J.Biol.Resp.Med. 8*:625-643 (1989)], which both inhibits proliferation of T_{H}2 cells and stimulates differentiation of T_{H}1 cells [Gajewski, *et al., J.Immunol. 143*:15-22 (1989); Gajewski, *et al., J.Immunol. 146*:1750-1758 (1991)]. The mechanism by which LPS potentiates the immune response is therefore through direct stimulation of B cells, and indirect regulation of both T_{H}1 and T_{H}2 cell populations.

Still other modes of immunopotentiation have been reported for other adjuvants. Oil emulsions (*i.e*., Freund's complete adjuvant [FCA], Freund's incomplete adjuvant [FIA]) and liposomes act through depot formation as does alum, thus allowing for slow release of antigen. Slow release of antigen permits extended exposure of the antigen to the immune system and also allows for initial immunization with a dosage of antigen that, if delivered at one time, would ordinarily be counterproductive to antibody formation. It has been previously reported that while a large initial dose of antigen results in the production of a higher immediate titer of antibody, the increase in antibody titer and increase in antibody specificity as a function of time is not as great as observed with lower and more frequent doses of antigen [Siskind, G., *Pharm.Rev. 25*:319-324 (1973)]. Therefore, adjuvants which control presentation of an antigen to the immune system modulate antigen dosage in addition to altering the form, or complexity, of the antigen.

To date, only one adjuvant, alum [AlK(SO₄)₂·H₂O], has proven sufficiently non-toxic to permit its use in humans. Alum not only acts through T_{H}2 cell activation, depot formation and slow release of antigen following immunization [Edelman, *Rev.Infect.Dis. 2*:370-383 (1980); Warren, *et al., Ann.Rev.Immunol. 4*:369-388 (1986)], but also through granuloma formation by attracting immunocompetent cells [White, *et al., J.Exp.Med. 102*:73-82 (1955)] and activation of complement [Ramanathan, *et al., Immunol. 37*:881-888(1979)]. However, alum is not without its negative side effects which include erythema, subcutaneous nodules, contact hypersensitivity, and granulomatous inflammation. Other adjuvants, which are widely employed outside of human application, are also the focus of continuing research to develop acceptable alternatives for use in humans. Included are the above mentioned oil emulsions (*i.e*., FCA and FIA), bacterial products (*i.e*., LPS, cholera toxin, mycobacterial components and whole killed *Corynebacterium parvum, Corynebacterium granulosum*, and *Bordetella pertussis*, liposomes, immunostimulating complexes (ISCOMs), and naturally occurring and derivatized polysaccharides from other than bacterial sources.

The immunopotentiating capacity of polysaccharides has been a focus of investigation over the past few years as these compounds are widespread in nature; *e.g*., as structural components in the cell walls of bacteria, and exoskeletons of insects and crustacea. Lipopolysaccharide (LPS) isolated from certain Gram-negative bacteria is one such polysaccharide even though the adjuvant properties of LPS are derived mainly from the lipid A region of the molecule, and not from the *o*-specific polysaccharide or core oligosaccharide regions of the molecule. LPS, which augments both humoral [Johnson, *et al*., *J.Exp.Med. 103*:225-246 (1956)] and cell-mediated immunity [Ohta. *et al., Immunobiology 53*:827 (1984)], possesses numerous biological activities, but is impractical for use in humans due to its inherent toxicity as reviewed by Gupta, *et al*., *Vaccine 11*:291-306 (1993). Attention has therefore shifted to other polysaccharides including, among others, chitosan.

Chitosan [β-(1-4)-2-amino-2-deoxy-D-glucan] is a derivative of chitin and has been widely used in biomedical applications, due in part to is biodegradability by lysozyme and low toxicity in humans. These same properties have resulted in increased interest in chitosan as an immunopotentiating agent. For example, Matuhashi, *et al.,* in U.S. Patent No. 4,372,883, disclosed conjugation of soluble polysaccharides, including chitosan, to normally toxic antigens, conjugation thereby detoxifying the antigen and permitting its use as an immunogen. Matuhashi *et al*., however, did not address the use of insoluble forms of chitosan, nor did Matuhashi compare the resulting serum antibody titer with that obtained from immunization with other known adjuvants.

Likewise, Suzuki, *et al*., in U.S. Patent 4,971,956, disclosed the use of water soluble chitosan-oligomers as therapeutics for treatment of bacterial and fungal infections, as well as for the treatment of tumors. Suzuki, *et al*, discussed the difficulty in modifying chitosan to produce an appropriate water soluble form, disclosing that water-insoluble forms are impractical for therapeutic application. In addition, Suzuki *et al*., does not disclose conjugation of an antigen to chitosan to effect enhanced immune response.

Mitsuhashi, *et al*., in U.S. Patent 4,814,169, disclosed the use of human protein conjugated to soluble polysaccharides, including chitosan, to generate antibodies against human protein in non-human animals. Administration of the human protein/polysaccharide solution was by intravenous, intraperitoneal, or subcutaneous injection. Other routes, including oral and rectal administration, were not addressed in the disclosure.

Nishimura, *et al.* [*Vaccine 2*:93-99 (1984)] reported the immunological properties of derivatives of chitin in terms of activation of peritoneal macrophages *in vivo*, suppression of tumor growth in mice, and protection against bacterial infection. Results suggested that both chitin and chitosan were ineffective stimulators of host resistance against challenge with tumor cells or bacteria, but that chitosan moderately induced cytotoxic macrophages. Results with modified, de-acetylated chitosan, which forms a gel in an aqueous environment, was shown to more effectively activate macrophages, suppress tumor growth and stimulate resistance to bacterial infection.

Marcinkiewicz, *et al.,* [*Arch.Immunol.Ther.Exp. 39*:127-132 (1991)] examined the immunoadjuvant activity of water-insoluble chitosan and reported significant enhancement of T-dependent humoral response, but only moderate augmentation of T-independent humoral response. The enhanced humoral response was detected with chitosan at doses of 100 mg/kg administered either intravenously or intraperitoneally. Subcutaneous and oral administration were specifically reported as being ineffective. In addition, Marcinkiewicz, *et al*., does not suggest conjugation of an antigen to insoluble chitosan, stating that chitosan "resulted in the same response irrespective of the site of administration either together or separately from antigen. "

In light of the fact that only one existing adjuvant has been approved for use in humans, there thus exists a need in the art to provide novel and less toxic adjuvants for potential application in humans. Improved adjuvants will permit the production of more effective vaccines and will improve the production of monoclonal antibodies with therapeutic potential.

### SUMMARY OF THE INVENTION

The present invention provides an adjuvant consisting essentially of in combination, chitosan with a chelated metal ion. The metal ion may, for example be iron or a transition metal such as copper, nickel, or zinc.

In another aspect, the invention provides for the use of the adjuvant in a method for potentiating an immune response comprising the steps of conjugating an antigen to the chitosan adjuvant and administering the protein/adjuvant conjugate to animal. The immune response is characterized by a rapid class switch from IgM to IgG production. The present invention encompasses antigens including antigens in the form of proteins, carbohydrates, lipids, glycoproteins or combinations thereof. The chitosan adjuvant may be soluble or insoluble; the insoluble adjuvant being either gelatinous or particulate in form. Conjugation of the antigen may be effected through chemical crosslinking, ionic interaction, physical intercalation, hydrophobic interactions, hydrophilic interactions, or covalent modification. A preferred method for crosslinking an antigen to the adjuvant is via use of glutaraldehyde. When glutaraldehyde is used for crosslinking, the resulting antigen/adjuvant immunogen may be either particulate or gelatinous, depending on the degree of crosslinking. The ratio of antigen to chitosan may range from 1:100 to 100:1, but is preferable in the range of 1:4 to 1:10, all ratios being weight of antigen to wet weight of chitosan. An alternative preferred ratio is 10:1. Antigen may be conjugated to the particulate chitosan adjuvant on the exterior surface of the particle, on an interior surface of the particle, or a combination of both interior and exterior conjugation. The antigen/adjuvant conjugate may be administered to the animal orally, intra-nasally, intra-vaginally, intra-rectally, or via intrdperitoneal, intravenous, or subcutaneous injection; administration may comprise a single route or a multiplicity of routes. The antigen/adjuvant conjugate may be administered alone, or in combination with any of a number of other adjuvants. Immunization may comprise a single administration or a multiplicity of administrations.

Also provided in an immunogen, the immunogen consisting essentially of chitosan with a chelated metal ion and an antigen. The antigen can include, but is not limited to proteins, carbohydrates, lipids, glycoproteins or combinations thereof. Protein antigens may be naturally occurring or recombinant. The antigen may be conjugated to the adjuvant by covalent bonding. Preferred covalent attachment is via crosslinking using glutaraldehyde. The resulting crosslinked immunogen may be particulate or gelatinous, depending on the degree of crosslinking. The antigen may also be combined with the adjuvant through ionic interaction. For example, when the antigen is a recombinant protein which comprises a poly-histidine (poly-HIS) amino acid sequence, the poly-HIS region can interact with the chelated metal ion. When the antigen is a protein, the poly-HIS amino acid sequence may be located at the carboxyl or amino terminus, or an internal region of the protein which does not significantly alter the naturally occurring tertiary or quanenary structure of the protein.

Also provided is a method for producing an adjuvant comprising the steps of preparing a chitosan solution, chelating a metal ion to the chitosan to produce a metal/chitosan complex, and isolating the metal/chitosan complex. Comprehended are methods which include a metal ion which may be, but is not limited to, iron or a transition metal such as copper, zinc, or nickel.

The invention also provides a method for producing an immunogen comprising the steps of preparing a chitosan solution, chelating a metal ion to the chitosan to produce a metal/chitosan complex, and combining an antigen with the metal/chitosan complex. The metal ion in the immunogen can include, but is not limited to, iron or a transition metal such as copper, nickel, or zinc. The antigen may be, but is not limited to a protein, carbohydrate, lipid, glycoprotein or combination thereof. The protein antigen may be naturally occurring or recombinant. The antigen may be combined to the adjuvant by covalent bonding. Preferred covalent attachment is effected by crosslinking. The preferred agent for crosslinking is glutaialdehyde. The resulting crosslinked immunogen may be particulate or gelatinous, depending on the degree of crosslinking. Alternatively, the antigen may be combined with the adjuvant via ionic interaction. Preferred ionic interaction is effected via a recombinant protein antigen comprising a poly-HIS amino acid sequence which interacts with the chelated metal ion. The poly-HIS sequence on the protein antigen may be located at either the carboxyl or amino terminus of the protein. Alternatively, the poly-HIS region may be located at an internal region of the protein which does not significantly alter the tertiary or quartenary structure of the naturally occurring protein. The resulting immunogen may be administered

Also provided by the invention is a method for producing an immunogen comprising the steps of preparing a chitosan solution, combining an antigen with the chitosan to form an antigen/chitosan complex, and chelating a metal ion to the antigen/chitosan complex. The metal in the immunogen may be, but is not limited to, iron or a transition metal, such as copper, nickel, or zinc. In the preferred method, the antigen is covalently attached to the chitosan via crosslinking. The preferred agent to effect the crosslinking is glutaraldehyde. The antigen may be, but is not limited to, a protein, carbohydrate, lipid, glycoprotein or combination thereof. If the antigen is a protein, it may be either naturally occurring or recombinant.

The invention provides for a method for potentiating an immune response comprising the steps of mixing an antigen with a chitosan adjuvant of the invention and administering the suspension to an animal. The immune response is characterized by a rapid class switch from IgM to IgG production. The ratio of antigen to chitosan may range from 1:100 to 100:1, but is preferable in the range of 10:1 to 1:10. The chitosan adjuvant may be soluble or insoluble; the insoluble adjuvant being particulate or gelatinous in form. The antigen/adjuvant suspension may be administered to the animal orally, intra-nasally, intro-vaginally, intra-rectally, or via intraperitoneal, intravenous, or subcutaneous injection; administration may comprise a single route or a multiplicity of routes. The antigen/adjuvant mixture may be administered alone, or in combination with other adjuvants. Immunization may comprise a single administration or multiple administrations.

As another aspect, the invention provides a method for potentiating an immune response comprising the steps of forming an adjuvant consisting essentially of chitosan and a chelated metal ion to form a metal/chitosan complex, combining an antigen with the metal/chitosan complex to form an immunogen, and administering the immunogen to an animal. The metal ion component of the immunogen may be, but is not limited to, iron or a transitional metal such as copper, nickel, or zinc. The antigen component of the immunogen may be, but is not limited to a protein, carbohydrate, lipid, or combination thereof. The weight:weight ratio of antigen to chitosan may be 100:1 to 1:100. A preferred ratio is 10:1, as determined by weight of antigen to wet weight of metal/chitosan complex. The antigen may be covalently bonded to the metal/chitosan complex; the preferred method for covalent attachment is via crosslinking. A preferred crosslinking agent is glutaraldehyde and the resulting crosslinked immunogen may be particulate or gelatinous depending on the degree of crosslinking. Alternatively, the antigen my be attached to the adjuvant via ionic interaction. A preferred method for attachment with ionic interaction is through use of a recombinant protein antigen comprising a poly-HIS amino acid sequence, the poly-HIS sequence be capable of interacting with the chelated metal ion. The poly-HIS region of the protein may be carboxyl or amino terminal to the protein, or may be internal to the protein in such a manner as to not significantly alter the naturally occurring tertiary or quaternary structure of the protein. The immunogen may be administered to the animal orally, intra-nasally, intra-vaginally, infra-rectally, or via intraperitoneal, intravenous, or subcutaneous injection; administration may comprise a single route or a multiplicity of routes. The immunogen suspension may be administered alone, or in combination with other adjuvants. Immunization may comprise a single administration or multiple administrations.

In another aspect of the invention, a composition is provided which, when administered to an animal, will potentiate an immune response, the composition comprising a conjugate between a protein and chitosan adjuvant of the invention. The chitosan component may be soluble or insoluble; the insoluble adjuvant being gelatinous or particulate in form, depending on the method by which the antigen is combined with the adjuvant. Conjugation may be effected through chemical crosslinking, ionic interaction, physical intercalation, or covalent attachment. The ratio of protein to chitosan may range from 1:100 to 100:1, but is preferable in the range of 1:4 to 1:10. The resulting composition is suitable for administration orally, intra-nasally, intra-vaginally, intra-rectally, or via intraperitoneal, intravenous, or subcutaneous injection.

In another aspect of the invention, a composition is provided which, when administered to an animal, will potentiate an immune response, the composition comprising a suspension of a protein and a chitosan adjuvant of the invention. The ratio of protein to chitosan may range from 1:100 to 100:1, but is preferable in the range of 1:4 to 1:10. The resulting composition is suitable for administration orally, intranasally, intravaginally, intrarectally, or via intraperitoneal, intravenous, or subcutaneous injection.

In another aspect of the invention, a method is provided for preparing a compound which potentiates an immune response, wherein an antigen is conjugated to a chitosan adjuvant, the resulting conjugate thereafter being suitable for administration to an animal. Conjugation may be effected through chemical crosslinking, ionic interaction, physical intercalation, hydrophobic interactions, hydrophilic interactions, or covalent attachment. The ratio of protein to chitosan may range from 1:100 to 100:1, but is preferable in the range of 1:4 to 1:10.

In another aspect of the invention, a method is provided for preparing a compound which potentiates an immune response, wherein an antigen is mixed with a chitosan adjuvant to form a suspension which can subsequently be administered to an animal. The ratio of protein to chitosan may range from 1:100 to 100:1, but is preferable in the range of 1:4 to 1:10.

### DESCRIPTION OF THE DRAWINGS

Numerous other aspects and advantages of the present invention will be apparent upon consideration of the following description thereof.

### DETAILED DESCRIPTION OF THE INVENTION

The present invention is illustrated by the following examples relating to a method of immunopotentiation which utilizes a protein/adjuvant conjugate or a protein/adjuvant suspension, as well as methods to prepare the conjugate and/or suspension. In particular, example 1 describes a general immunization protocol. Example 2 relates to an enzyme linked immunosorbent assay (ELISA) utilized in all measurements to determine serum antibody titer. Example 3 relates preparation of a metal/chitosan complex adjuvant. Example 4 addresses immunopotentiation with a porcine zona pellucida protein combined with various metal/chitosan adjuvants. Example 5 demonstrates use of a zinc/chitosan adjuvant with another porcine zona pellucida protein. Example 6 describes preparation of an iron/chitosan adjuvant. Example 7 addresses palmitylation of a metal/chitosan complex. Example 8 describes the immune response with and without palmitylation of the metal/chitosan adjuvant.

### Example 1

### Immunization Protocol

Unless otherwise stated in the examples, the following immunization protocol was employed throughout.

Mice were immunized generally with variable amounts of antigen in a 100 µl volume either via intraperitoneal, subcutaneous or intramuscular injection with desired recombinant protein complexed with metal chitosan. Prior to the initial immunization, animals were bled to obtain control serum. After the initial immunization, animals were bled at weekly intervals and antigen titer determined by ELISA as described in Example 2. All animals were boosted with an injection identical to the initial immunogen three to four weeks after the initial immunization.

### Example 2

### Enzyme Linked Immunosorbent Assay (ELISA)

Assessment of the degree to which chitosan, in combination with an antigen, is able to enhance an immune response was made utilizing an enzyme linked immunosorbent assay (ELISA) well known in the art. The protocol employed was as follows.

Flat bottom polyvinyl chloride 96 well assay plates (Falcon #3912) were coated overnight at 4°C with antigen (50 µl/well at 5-10 µg/ml) in carbonate-bicarbonate buffer, pH 9.6. The plates were washed with PBS containing 0.5% polyoxyethylene (20) sorbitan monolaurate (Tween 80) (Mallinckrodt Specialty Chemical Company, Paris KY) (PBS-T) and postcoated with PBS-T containing 2% non-fat dry milk (PBS-NFDM) for two hours at room temperature to inhibit nonspecific binding. Following three washes with PBS-T, 50 µl of sample or control serum diluted in PBS-T was added to each well, the plates were covered and incubated for 1 hr at room temperature. The plates were emptied, washed three times with PBS-T and 50 µl of a 1:1000 dilution of biotin labelled goat anti-mouse IgG (including both heavy and light polypeptide chains) (Zymed Laboratories, South San Francisco, CA) or biotin labelled goat anti-mouse IgM (Southern Biotechnology Associates, Birmingham, AL) in PBS-T was added to each well. After 1 hr at room temperature the plates were washed with PBS-T and 50 µl of PBS-T containing horseradish peroxidase conjugated avidin biotin complex from a Vectastain ABC kit (Vector Laboratories, Inc., Burlingame, CA). The enzyme substrate was 50µl of 400 µg/ml *o*-phenylenediamine (Sigma). Plates were allowed to develop in the dark at room temperature for 20 min. The optical density (OD) at 450 nm was read on a Dynatec Laboratories MR 700 (Chantilly, VA), using water as a blank.

### Example 3

### Preparation of Metal/Chitosan Complexes Containing Either Zinc, Copper, or Nickel

Previous observations during the purification of poly-HIS-tagged zona pellucida (ZP) proteins using nickel (Ni)-Sepharose chromatography suggested that sperm readily bound to the Ni-immobilized ZP proteins as compared to the binding detected with ZP proteins immobilized on other affinity columns. This observation is of particular interest because ZP proteins play a role in sperm binding to oocytes. This observation suggested that the poly-HIS-tagged ZP proteins may be capable of maintaining a more natural conformation when immobilized by this method. This observation, when combined with a previous observation that chitin possesses the ability to chelate transition metal ions, led to an investigation of the possible use of a metal/chitosan chelate for binding a poly-HIS-tagged antigen and its use as an immunopotentiator. Given that transition metal ions are toxic in general, several different ions were initially tested to produce the metal/chitosan complexes.

To prepare chitosan/metal complex adjuvants containing either zinc, copper or nickel, a 2% chitosan solution was initially prepared by dissolving 2 g chitosan (CTC Organics, Atlanta, GA) in 100 ml 2% acetic acid, the resulting solution sterilized by autoclaving. As an alternative, the chitosan solution can also be prepared by dissolving 2 g in 100 ml 0.5 M sodium acetate pH 4.5. A zinc acetate, nickel sulfate or copper sulfate solution was prepared in deionized water at a molarity between 0.001 to 0.2 M and filter sterilized. The 2% chitosan solution was diluted 1:1 using deionized water and 4 ml of the resulting 1% chitosan solution was added to 10 ml of the desired metal salt solution. The resulting suspension was mixed on an end to end shaker for 2 to 4 hours at room temperature. The mixture was sonicated using a Branson Sonifier 250 for 3 to 5 minutes and the pH of the mixture adjusted to 12.0 - 12.5 with 10 N NaOH during sonication. When the zinc salt was employed, a white complex precipitate was formed, when the nickel salt was used, the complex was light green and when the copper salt was used, the complex was blue. After sonication, the mixture was centrifuged at 2000 rpm (1000 x *g*) for 10 minutes and supernatant discarded. The pellet was washed twice with PBS, pH 7.2, centrifuged after each wash, the wet weight of the pellet determined, and the metal/chitosan complex pellet resuspended in 8 M urea, pH 7.8 to 8.0. The metal/chitosan complexes were either immediately coupled to an antigen, or stored in either 8 M urea or PBS at room temperature. The stored metal/chitosan complexes have shown to be stable for up to six months when stored by this method.

Antigens were associated with the metal/chitosan complex by one of two alternative methods. In the first method, proteins were crosslinked to the metal/chitosan complex using glutaraldehyde as previously described in Example 13 and the following example. In another method, (described in this example), recombinant proteins contained a C-terminal poly-histidine region to facilitate purification and to permit association of the protein with the metal component of the metal/chitosan complex. This procedure is described as follows.

Recombinant proteins modified to include six histidines (poly-HIS) were expressed in either bacteria, yeast, insect, or CHO cells and purified using Ni-Sepharose or cobalt (Co)-Sepharose chromatography both of which are well known in the art. Recombinant protein was equilibrated in 8 M urea and incubated with the chitosan metal complex in a plastic tube for 1 to 3 hours at room temperature. Following incubation, protein/metal/chitosan complex was pelleted by centrifugation for 10 minutes at 1000 x *g* and the amount of complex bound protein estimated by determining protein concentration in the supernatant following centrifugation and subtracting the amount initially added to the binding reaction. In general, the resulting ratio of mg antigen:mg wet weight metal/chitosan averaged 10:1. The pellet was washed two times with PBS, resuspended in PBS and the concentration adjusted to 1 mg antigen/ml buffer.

Specific use and results from the use of an antigen/zinc/chitosan as an immunogen are described below in Examples 4 and 5.

### Example 4

### Immune response To Recombinant Pig Zona Peliucida Protein B (Pig-b) Complexed With Chitosan Complexed With Either of Nickel, Copper or Zinc

Recombinant porcine zona pellucida protein pig-b, expressed in bacteria, was complexed to either nickel/chitosan, copper/chitosan, or zinc/chitosan as described above in Example 3. Individual groups of mice were immunized intraperitoneally with either 5, 25, 100 or 250 µg protein/mouse in case of nickel/chitosan and zinc/chitosan, or either 5 or 25 µg protein/mouse in case of copper/chitosan. Each group received a booster immunization on day 21 after the initial immunization with the same amount of antigen and metal/chitosan. Mice were bled on day 8, 21, 28 and 49 after the first immunization and serum titer determined by ELISA as described above in Example 2. Serum titers determined against bacterially expressed zona pellucida pig b and heat solubilized total native pig zona are shown in Tables 1 and 2, respectively. These results suggest little difference between the chelate adjuvants in terms of immunopotentiation.

**Table 1**

| **Serum Titer Determined By ELISA Using Recombinant Pig-C** | | | | |
|---|---|---|---|---|
| Dose/mice (µg)- | 5 | 25 | 100 | 250 |
| Pig-B Nickel-chitosan | | | | |
| Day | | | | |
| 8 | 200 | 100 | 800 | 3.2K |
| 21 | 4k | 16k | 32k | 8k |
| 28 | 32k | 64k | >64k | >64k |
| 49 | 16k | 64k | >64k | >64k |

| Pig-B Zinc-chitosan | | | | |
|---|---|---|---|---|
| Day | | | | |
| 8 | 200 | 100 | 800 | 3.2K |
| 21 | 16k | 32k | 64k | >64k |
| 28 | >64k | >64k | >64k | >64k |
| 49 | 32k | >64k | >64k | >64k |

| Pig-B Copper-chitosan | | | | |
|---|---|---|---|---|
| Day | | | | |
| 8 | 400 | 200 | ND* | ND* |
| 21 | 2k | 2k | ND* | ND* |
| 28 | 32k | 32K | ND* | ND* |

| | | | | |
|---|---|---|---|---|
| *ND- dose not done | | | | |

**Table 2**

| Serum Titer Determined By ELISA Using Heat-Denatured Total Zona Pellucida Protein | | | | |
|---|---|---|---|---|
| Dose/mice (µg)- | 5 | 25 | 100 | 250 |
| Pig-B Nickel-chitosan | | | | |
| Day | | | | |
| 8 | 200 | 200 | 800 | 400 |
| 21 | 500 | 1k | 4k | 4k |
| 28 | 4k | 8k | 16k | 16k |

| Pig-B Zinc-chitosan | | | | |
|---|---|---|---|---|
| Day | | | | |
| 8 | 200 | 200 | 200 | 3.2 |
| 21 | 500 | 2k | 32k | 8k |
| 28 | 8k | 16k | >64k | >64k |

| Pig-B Copper-chitosan | | | | |
|---|---|---|---|---|
| Day | | | | |
| 8 | 800 | 1.6k | ND* | ND* |
| 21 | 500 | 1k | ND* | ND* |
| 28 | 1k | 4k | ND* | ND* |

| | | | | |
|---|---|---|---|---|
| *ND- dose not done | | | | |

### Example 5

### Immunization With Bacterially Expressed Zona Pellucida Pig-C Protein Complexed with Zinc/Chitosan

Balb/c mice were immunized with bacterially expressed recombinant pig zona pellucida C protein (pig-c) and complexed to zinc/chitosan by the following protocol.

The pig-c antigen was initially expressed in and purified from *E.coli* and subsequently complexed with zinc-chitosan prepared as described above in Example 3. Mice were immunized intraperitoneally with 100 µg antigen/mouse (Group I). In one control group (Group II), mice were immunized at 100 µg/mouse with purified protein alone, and in another control group (Group III), mice were immunized with pig-c protein associated with precipitated zinc acetate following the same protocol in Example 3 for making zinc/chitosan except that 1% chitosan was not added to the solution. The mice were boosted on day 21 with pig-c antigen at 100 µg/mouse complexed with zinc/chitosan by the same preparation used for the initial immunization. Mice were bled on day 21, 28 and 49 by retro-orbital puncture and serum titers were measured by ELISA as described in Example 2. Results are shown in Table 3.

On day 21, serum titer in Group I averaged 64k as compared to titers of less than 250 for Group II and 1k for Group m. After a booster immunization on day 21 following the initial immunization, serum titers determined from bleeds taken on day 28 were found to be more than 64k for Group I, and 4k for both Groups II and III. These data indicate that neither the antigen alone, nor the metal/antigen components of the adjuvant are responsible for the observed immune response, but that the immune response requires the appropriate antigen/metal/chitosan complex for rapid stimulation of an immune response that persists over time.

**Table 3**

| Comparison of Immune Response Using Antigen/Metal/Chitosan, Antigen or Antigen/Metal | | | |
|---|---|---|---|
| Days After Immunization | Antigen | | |
| | Recombinant Pig-C on Zinc/Chitosan (Group I) | Recombinant Pig-C (Group II) | Recombinant Pig-C Precipitated on Zinc (Group III) |
| 21 | 32k | <250k | 1k |
| 28 | >64k | 8k | 4k |
| 49 | >64k | 4k | 4k |

### Example 6

### Preparation of Chitosan-Metal Complexes Containing Iron

For preparation of an iron/chitosan complex immunogen, 4 g ferric ammonium citrate was dissolved in 10 ml distilled water with 100 µl 1.6 N HCl. Four ml of the 1% chitosan solution prepared as described above was sonicated and 200 µl of the ferric ammonium citrate solution was added during sonication. The resulting solution was centrifuged and the pellet containing iron/chitosan complex was washed once in deionized water. Recombinant proteins modified to include six histidine residues were coupled the iron/chitosan complex as above except 8 M urea was not added. Alternatively, with proteins such as egg ovalbumin or bacterial/viral proteins which did not include the poly-histidine sequence, the antigen was coupled to the iron/chitosan complex by one of the methods described as follows.

In a first method, iron/chitosan complex was prepared as described above. A protein solution in PBS pH 7.4 was added and the resulting suspension vortexed to obtain a uniform mixture. A 25 % glutaraldehyde solution was diluted 1:4 with PBS and added to the antigen/chitosan mixture at a volume of 20 µl per ml of the antigen/chitosan mixture. The resulting suspension was mixed on an end to end shaker for at least 4 hours. After mixing, 100 µl 1 M glycine in PBS was added to neutralize unreacted glutaraldehyde and incubation continued for 1 hour at room temperature. The mixture was centrifuged for 10 minutes at 1000 x *g* and the resulting pellet washed two times with PBS. After the final wash, the pellet was resuspended at a antigen concentration of 1 mg/ml in PBS and immediately used to immunize animals.

In an alternative to the preceding protocol, 1% chitosan, prepared as described above and not complexed with iron, was added to a desired protein and the resulting mixture shaken gently to prepare a uniform mixture. As described above, a 25% glutaraldehyde solution was diluted 1:4 with PBS and added to the antigen/chitosan mixture at a volume of 20 µl per ml antigen/chitosan mixture. The resulting solution was incubated at room temperature for approximately 2 to 6 hours to permit limited glutaraldehyde-induced crosslinking, after which time the mixture became a gel. The loosely crosslinked material was generally clear, flowable and did not appear to be particulate when viewed under a microscope. The resulting material was sonicated for 2-3 minutes during which time 400 µl of 4 g/ml ferric ammonium citrate solution was added which induced formation of fine yellowish-brown particles in which the antigen was entrapped. The antigen/iron/chitosan particles were pelleted by centrifugation for 10 minutes at 1000 x *g* and the resulting pellet resuspended at an antigen concentration of about 1 mg/ml. This solution was used to immunize mice by the protocol described in Example 1.

### Example 7

### Preparation of Palmitylated Iron Chitosan

In view of previous reports regarding immune stimulating complex (ISCOM) which have been demonstrated to induce a higher titer when the complex is palmitylated, the following procedure was carried out to modify the antigen/metal/chitosan complex prior to immunization.

Protein antigen was incorporated into iron chitosan particles by any of the method described above and palmitic groups were added by the following procedure. Twenty mg palmitic anhydride was dissolved in 1 ml of triethylamine and the solution added to a protein/metal/chitosan complex solution at a ratio of 10 µl palmitic anhydride for every 1 mg of antigen in the iron/chitosan complex solution. The resulting solution was mixed by gentle tapping and allowed to sit at room temperature for at least two hours before the preparation was diluted and used for immunizations.

### Example 8

### Immunization With Pig-C Incorporated In Iron/chitosan Particles With And Without Palmitylation

Purified zona pellucida pig-C protein expressed in *E.coli* was incorporated onto iron/chitosan particles as follows. Ten ml 1% chitosan solution in 2 % acetic acid was sonicated 2-3 minutes with continuous addition of 400 µl 0.4 g/ml ferric ammonium citrate solution during sonication. Resulting iron/chitosan particles were pelleted by centrifugation and the pellet washed two times with deionized water. A solution containing pig-c protein expressed in *E.coli* was adjusted to a pH 7.8 with PBS and incubated with iron/chitosan particles, incubation was allowed to continue overnight with mixing on an end to end shaker. After incubation, the antigen/metal/chitosan particles were pelleted by centrifugation at 1000 x *g* for 10 minutes and protein concentration in supernatant measured by the Bradford method known in the art. The final ration of antigen to metal/chitosan was determined to be 3.7 mg pig-c protein bound per gram wet weight iron-chitosan. Prior to use as immunogen, the pig-c iron was adjusted to a concentration of 1 mg antigen/ml.

In order to produce a palmitylated pig-c/iron/chitosan immunogen, particles produced as described above were initially incubated in 8 M urea. A solution of 20 mg palmitic anhydride was prepared in 1 ml triethylamine, and 10 µl of the solution was added to 1 mg equivalent (protein) pig-c/iron/chitosan. The mixture was shaken gently and allowed to incubate at room temperature for at least 2 hours before use as an immunogen.

Balb/c mice were immunized intraperitoneally with either pig-c incorporated in iron/chitosan particles or palmitylated pig-c/iron/chitosan particles. Individual groups of mice were initially immunized with 100 µg protein and each group was booted on day 22 following the initial immunization with identical dosages and preparations. Mice were bled on day 7, 14, 22, 42, 56 and 63 after the initial immunization and serum antibody titers estimated by ELISA against recombinant pig-c protein. A comparison of the serum titers against pig-c in the both groups are summarized in the Table 4.

Serum titers were comparable in the primary response, but after the booster immunization, serum titer in the group of mice injected with palmitylated antigen was significantly higher (> 128k) that determined for the non-palmitylated group (64k).

**Table 4**

| Effect of Palmitylation on Metal/Chitosan Immunopotentiation | | |
|---|---|---|
| Days After Immunization | Preparation | |
| | Pig-C/ Iron/Chitosan | Palmitylated Pig-C/ Iron/Chitosan |
| 7 | 400 | 400 |
| 14 | 3.2k | 3.2k |
| 22 | 3.2k | 3.2k |
| 42 | 64k | 128k |
| 56 | 128k | >128k |
| 63 | 64 | >128k |
| 76 | 64k | >128k |

## Claims

1. An adjuvant consisting essentially of, in combination, chitosan and a chelated metal.

2. The adjuvant of claim 1 wherein the chelated metal is a transition metal.

3. The adjuvant of claim 2 wherein-the transition metal is copper, zinc or nickel.

4. The adjuvant of claim 2 wherein the chelated metal is iron.

5. An immunogen consisting essentially of an antigen in combination with the adjuvant of any of claims 1 to 4.

6. The immunogen of claim 5 wherein the antigen is covalently cross-linked to the adjuvant.

7. The immunogen of claim 6 wherein cross-linking is effected using glutaraldehyde.

8. The immunogen of claim 6 or claim 7 which is particulate.

9. The immunogen of claim 6 or claim 7 which is gelatinous.

10. The immunogen of any of claims 5 to 9 wherein the antigen is a protein.

11. The immunogen of claim 10 wherein the protein comprises a poly-histidine amino acid sequence.

12. The immunogen of claim 11 wherein the poly-histidine amino acid sequence is linked to the carboxyl terminus of the protein.

13. The immunogen of claim 11 wherein the poly-histidine amino acid sequence is linked to the amino terminus of the protein.

14. The immunogen of any of claims 11 to 13 wherein the protein is attached to the adjuvant through interaction of the poly-histidine amino acid sequence and the chelated metal.

15. A method for producing an adjuvant according to any of claims 1 to 4, comprising preparing a chitosan solution and chelating a metal ion to the chitosan to form a metal/chitosan complex.

16. A method according to claim 15 further comprising isolating the metal/chitosan complex.

17. A method according to claim 15 or claim 16 further comprising combining an antigen with the metal/chitosan complex to form an immunogen.

18. A method according to claim 15 or claim 16 further comprising combining an antigen with the chitosan in the solution prior to chelating the metal ion to the chitosan to form an immunogen.

19. A method according to claim 17 or claim 18 wherein the immunogen is in accordance with any of claims 5 to 14.

20. The use of an antigen combined with an adjuvant consisting essentially of, in combination, chitosan and a chelated metal in the manufacture of a medicament for administration to an animal to potentiate an immune response **characterised by** rapid immunoglobulin class switching from production of IgM to IgG.

21. The use of claim 20 wherein the antigen is conjugated to the chitosan.

22. The use of claim 20 or claim 21 wherein the chitosan is an insoluble particulate.

23. The use of any preceding claim wherein the medicament is for intra-rectal administration.

24. The use of any of claims 20 to 23 wherein the medicament is for intra-peritoneal injection.

25. The use of any of claims 20 to 23 wherein the medicament is for subcutaneous injection.

26. The use of any of claims 20 to 25 wherein a weight:weight ratio of antigen:chitosan is in the range of 1:100 to 100:1.

27. The use of claim 26 wherein the weight:weight ratio of antigen to chitosan is in the range of 1:4 to 1:10.

28. The use of any one of claims 20 to 27 wherein the chelated metal is a transition metal.

29. The use of claim 28 wherein the transition metal is copper, zinc or nickel.

30. The use of any one of claims 20 to 27 wherein the chelated metal is iron.

## Patentansprüche

1. Adjuvans, das im Wesentlichen aus einer Kombination aus Chitosan und einem chelatierten Metall besteht.

2. Adjuvans nach Anspruch 1, worin das chelatierte Metall ein Übergangsmetall ist.

3. Adjuvans nach Anspruch 2, worin das Übergangsmetall Kupfer, Zink oder Nickel ist.

4. Adjuvans nach Anspruch 2,worin das chelatierte Metall Eisen ist.

5. Immunogen, das im Wesentlichen aus einem Antigen in Kombination mit einem Adjuvans nach einem der Ansprüche 1 bis 4 besteht.

6. Immunogen nach Anspruch 5, worin das Antigen kovalent mit dem Adjuvans vernetzt ist.

7. Immunogen nach Anspruch 6, worin das Vernetzen unter Einsatz von Glutaraldehyd erfolgt ist.

8. Immunogen nach Anspruch 6 oder 7, das teilchenförmig ist.

9. Immunogen nach Anspruch 6 oder 7, das gelatineartig ist.

10. Immunogen nach einem der Ansprüche 5 bis 9, worin das Antigen ein Protein ist.

11. Immunogen nach Anspruch 10, worin das Protein eine Polyhistidin-Aminosäuresequenz umfasst.

12. Immunogen nach Anspruch 11, worin die Polyhistidin-Aminosäuresequenz an den Carboxylterminus des Proteins gebunden ist.

13. Immunogen nach Anspruch 11, worin die Polyhistidin-Aminosäuresequenz an den Aminoterminus des Proteins gebunden ist.

14. Immunogen nach einem der Ansprüche 11 bis 13, worin das Protein an das Adjuvans durch Wechselwirkung der Polyhistidin-Aminosäuresequenz mit dem chelatierten Metall gebunden ist.

15. Verfahren zur Herstellung eines Adjuvans nach einem der Ansprüche 1 bis 4, das die Herstellung einer Chitosan-Lösung und das Chelatieren eines Metallions an das Chitosan zur Bildung eines Metall/Chitosan-Komplexes umfasst.

16. Verfahren nach Anspruch 15, das weiters das Isolieren des Metall/Chitosan-Komplexes umfasst.

17. Verfahren nach Anspruch 15 oder 16, das weiters das Kombinieren eines Antigens mit dem Metall/Chitosan-Komplex zur Bildung eines Immunogens umfasst.

18. Verfahren nach Anspruch 15 oder 16, das weiters das Kombinieren eines Antigens mit dem Chitosan in der Lösung vor dem Chelatieren des Metallions an das Chitosan zur Bildung eines Immunogens umfasst.

19. Verfahren nach Anspruch 17 oder 18, worin das Immunogen ein Immunogen nach einem der Ansprüche 5 bis 14 ist.

20. Verwendung eines Antigens in Kombination mit einem Adjuvans, das im Wesentlichen aus einer Kombination aus Chitosan und einem chelatierten Metall besteht, zur Herstellung eines Medikaments zur Verabreichung an ein Tier, um eine Immunreaktion zu verstärken, die durch eine rasche Umstellung der Immunglobulin-Klassen-Produktion von IgM auf IgG **gekennzeichnet** ist.

21. Verwendung nach Anspruch 20, worin das Antigen an das Chitosan konjugiert ist.

22. Verwendung nach Anspruch 20 oder 21, worin das Chitosan ein unlösliches Teilchenmaterial ist.

23. Verwendung nach einem der vorangegangenen Ansprüche, worin das Medikament zur intrarektalen Verabreichung dient.

24. Verwendung nach einem der Ansprüche 20 bis 23, worin das Medikament zur intraperitonealen Injektion dient.

25. Verwendung nach einem der Ansprüche 20 bis 23, worin das Medikament zur subkutanen Injektion dient.

26. Verwendung nach einem der Ansprüche 20 bis 25, worin das Gewichtsverhältnis zwischen Antigen und Chitosan im Bereich von 1:100 bis 100:1 liegt.

27. Verwendung nach Anspruch 26, worin das Gewichtsverhältnis zwischen Antigen und Chitosan im Bereich von 1:4 bis 1:10 liegt.

28. Verwendung nach einem der Ansprüche 20 bis 27, worin das chelatierte Metall ein Übergangsmetall ist.

29. Verwendung nach Anspruch 28, worin das Übergangsmetall Kupfer, Zink oder Nickel ist.

30. Verwendung nach einem der Ansprüche 20 bis 27, worin das chelatierte Metall Eisen ist.

## Revendications

1. Adjuvant consistant essentiellement, en combinaison, en chitosan et un métal chélaté.

2. Adjuvant de la revendication 1 où le métal chélaté est un métal de transition.

3. Adjuvant de la revendication 2 où le métal de transition est cuivre, zinc ou nickel.

4. Adjuvant de la revendication 2 où le métal chélaté est fer.

5. Immunogène consistant essentiellement en un antigène en combinaison avec l'adjuvant de l'une quelconque des revendications 1 à 4.

6. Immunogène de la revendication 5 où l'antigène est réticulé de manière covalente à l'adjuvant.

7. Immunogène de la revendication 6 où la réticulation est effectuée en utilisant de glutaraldéhyde.

8. Immunogène de la revendication 6 ou de la revendication 7 qui est particulaire.

9. Immunogène de la revendication 6 ou de la revendication 7 qui est gélatineux.

10. Immunogène de l'une quelconque des revendications 5 à 9 où l'antigène est une protéine.

11. Immunogène de la revendication 10 où la protéine comprend une séquence d'acides aminés de poly-histidine.

12. Immunogène de la revendication 11 où la séquence d'acides aminé de poly-histidine est enchaînée au carboxyle terminal de la protéine.

13. Immunogène de la revendication 11 où la séquence d'acides aminés de poly-histidine est enchaînée à l'amino terminal de la protéine.

14. Immunogène de l'une quelconque des revendications 11 à 13 où la protéine est attachée à l'adjuvant par interaction de la séquence d'acides aminés de la poly-histidine et du métal chélaté.

15. Méthode de production d'un adjuvant selon l'une quelconque des revendications 1 à 4 consistant, à préparer une solution de chitosan et à chélater un ion métallique au chitosan pour former un complexe métal/chitosan.

16. Méthode selon la revendication 15 consistant de plus à isoler le complexe métal/chitosan.

17. Méthode selon la revendication 15 ou la revendication 16 comprenant de plus la combinaison d'un antigène au complexe métal/chitosan pour former un immunogène.

18. Méthode selon la revendication 15 ou la revendication 16 consistant de plus à combiner un antigène au chitosan dans la solution avant de chélater l'ion métallique au chitosan pour former un immunogène.

19. Méthode selon la revendication 17 ou la revendication 18 ou l'immunogèn est selon l'une quelconque des revendications 5 à 14.

20. Utilisation d'un antigène combiné à un adjuvant consistant essentiellement en, en combinaison, du chitosan et un métal chélaté dans la fabrication d'un médicament pour administration à un animal pour potentialiser une réponse immune, **caractérisée par** le changement rapide de classe d'immunoglobuline de la production de IgM à IgG.

21. Utilisation de la revendication 20 où l'antigène est conjugué au chitosan.

22. Utilisation de la revendication 20 ou de la revendication 21 ou le chitosan est une matière particulaire insoluble.

23. Utilisation de toute revendication précédente où le médicament est pour administration intra-rectale.

24. Utilisation de l'une quelconque des revendications 20 à 23 où le médicament est pour injection intra-péritonéale.

25. Utilisation de l'une quelconque des revendications 20 à 23 où le médicament est pour injection sous-cutanée.

26. Utilisation de l'une quelconque des revendications 20 à 25 ou un rapport poids : poids d'antigène : chitosan est dans la gamme de 1 : 100 et de 100 : 1.

27. Utilisation de la revendication 26 où le rapport poids : poids de l'antigène au chitosan est dans la gamme de 1 : 4 à 1 : 10.

28. Utilisation de l'une quelconque des revendications 20 à 27 où le métal chélaté est un métal de transition.

29. Utilisation de la revendication 28 où le métal de transition est cuivre, zinc ou nickel.

30. Utilisation de l'une quelconque des revendications 20 à 27 où le métal chélaté est fer.
